Europäisches Patentamt

European Patent Office

Office européen des brevets

(11) Publication number: **0 209 994**
**A1**

# (12) EUROPEAN PATENT APPLICATION

(21) Application number: 86304909.4

(22) Date of filing: 25.06.86

(51) Int. Cl.4: **G01N 33/48** , G01N 33/543 , A61J 7/00

(30) Priority: 01.07.85 US 751359

(43) Date of publication of application:
28.01.87 Bulletin 87/05

(84) Designated Contracting States:
AT BE CH DE FR GB IT LI LU NL SE

(71) Applicant: **HYBRITECH INCORPORATED**
**11095 Torreyana Road**
**San Diego California 92126(US)**

(72) Inventor: **Donneux, Jacques**
**19 rue de l'Eglise**
**Chardeneux 5418(BE)**
Inventor: **Van de Winckel, Guy Charles-Louis**
**55 Bois Imperial de Rognac**
**Neupre 4121(BE)**
Inventor: **Thillon, Francoise Alberte Jeanne**
**29 rue de Centre**
**Tihange 5201(BE)**

(74) Representative: **Tapping, Kenneth George et al**
**Erl Wood Manor**
**Windlesham Surrey, GU20 6PH(GB)**

(54) Diagnostic assay bead dispenser.

(57) An apparatus is provided for dispensing into containers beads suitable for diagnostic assays. A trigger arrangement (14) having a first (31) and a second (30) gate member controls the dispensing of beads (21).

Fig. 3

## DIAGNOSTIC ASSAY BEAD DISPENSER

The present invention relates to dispensers used for dispensing into containers diagnostic assay beads suitable for diagnostic or immunologic assays.

Certain immunologic and diagnostic assays require the use of solid supports (hereinafter "beads") upon which certain chemical and/or biochemical reactions take place. Beads can be fashioned out of glass, metal or plastics.

Prior to the present invention, the dispensing of beads into reaction vessels was accomplished either by individually ladling the beads from a source container to the reaction vessel by use of forceps or similar devices. Although effective, this process is slow and time consuming. Difiglio, et al., U.S. Patent No. 4,101,284 describe a Multiple Bead Dispenser for Diagnostic Assay wherein a device is aligned with mating receptacles in a multi-well container and a plurality of beads are simultaneously dispensed into the multi-well container suitable for the diagnostic assays. While suited for its intended purpose, the device of Difiglio, et al. is not flexible in that it requires the reaction vessels to be in a particular alignment with one another prior to the dispensing of the beads. Furthermore, a constant number of beads are dispensed even though the number of reaction vessels may be different from the number of beads dispensed.

Accordingly, the present invention provides a lightweight hand-held apparatus for dispensing beads used as solid supports in immunologic diagnostic assays.

In particular, the present invention is directed to an apparatus for individually dispensing into reaction vessels beads used as solid supports in immunologic diagnostic assays. To this end, an apparatus having a bead container and a trigger mechanism wherein a trigger member operatively connected to a first gate member and a second gate member allows beads to be dispensed from the bead container one at a time.

More particularly there is provided an apparatus useful for dispensing diagnostic assay beads, which comprises in combination a body having a cavity, a bead chamber within said cavity, a barrel member having a first end and a second end, said barrel member defining a passage, said first end mounted within said cavity and in communication with said bead chamber, said second end extending outwardly from said body and including an orifice; a dispensing member comprising a first gate member and a second gate member, said first gate member positioned apart from said second gate member to allow a single bead to occupy the space between said first gate member and said second gate member; a trigger member projecting form said body and operatively connected to said body, said trigger member operatively connected to said dispensing member, said trigger member having a first trigger position and a second trigger position wherein when said trigger member is in said first trigger position said first gate member does not impede the travel of beads within said barrel member toward said orifice and said second gate member impedes the travel of beads within said barrel member toward said orifice; and wherein when said trigger member is in said second trigger position said first gate member impedes the travel of beads within said barrel member toward said orifice and said second gate member does not impede the travel of beads within said barrel member toward said orifice; biasing means biasing said trigger member toward said first trigger position.

Accordingly, the present invention provides a novel apparatus for dispensing beads used as solid supports in immunologic diagnostic assays. Other details and advantages will appear in the discussion below.

Figure 1 is a side elevation of the bead dispenser showing the dispenser as it would be positioned for use above a reaction vessel.

Figure 2 is a front view of the bead dispenser.

Figure 3 is a cross-sectonal view of the bead dispenser taken along lines 3-3 of Figure 2 showing the trigger mechanism in the first trigger position.

Figure 4 is a cross-sectional view of the bead dispenser showing the trigger mechanism in the second trigger position.

Figure 5 is a cross-sectional view of the barrel of the bead dispenser taken along lines 5-5 of Figure 3.

The diagnostic assay bead dispenser has been designed to be a lightweight apparatus for individually dispensing into reaction vessels single beads used as solid supports for immunologic diagnostic assays. The body of the bead dispenser may be fashioned from injection molded synthetic thermoplastics.

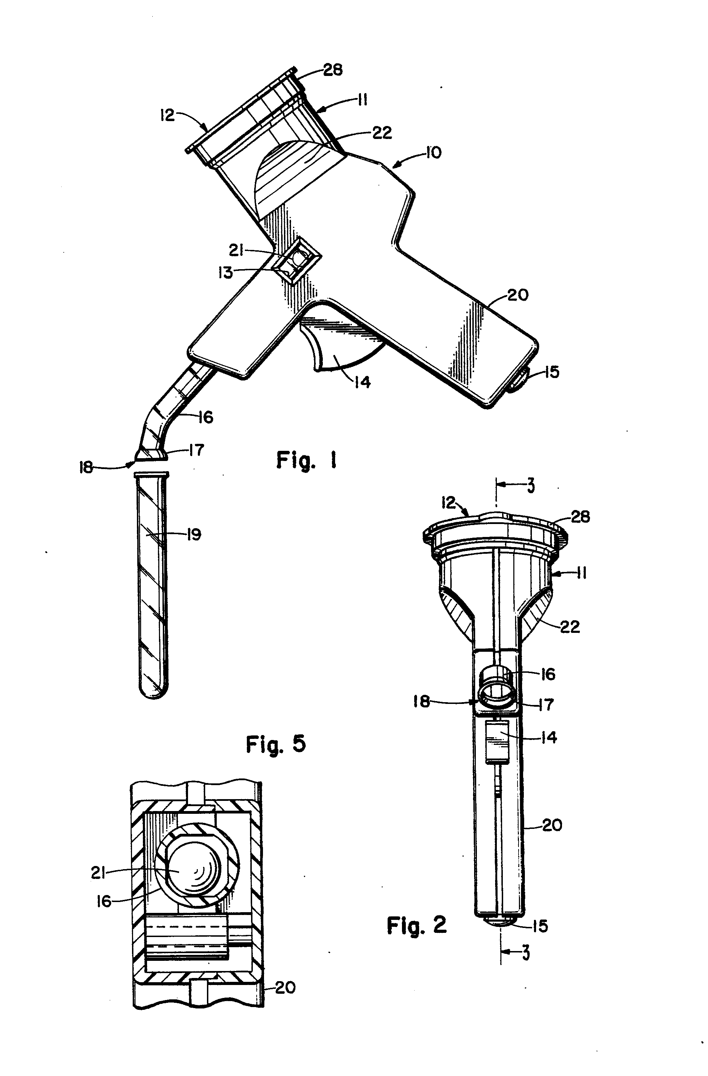

Turning in detail to the drawings, Fig. 1 illustrates a hand-held diagnostic bead dispenser 10 - (hereinafter referred to as "bead dispenser") positioned over a reaction vessel 19 into which a bead may be dispensed. As can be seen in the Figures, a single bead 21 is dispensed by actuation of trigger member 14 which is pivotally attached to the body at a pivot point 27. The bead is released into barrel member 16, which is curved so as to point generally downward, and drops out of the flared orifice 17 through the outboard end 18 of the barrel member and into reaction vessel 19.

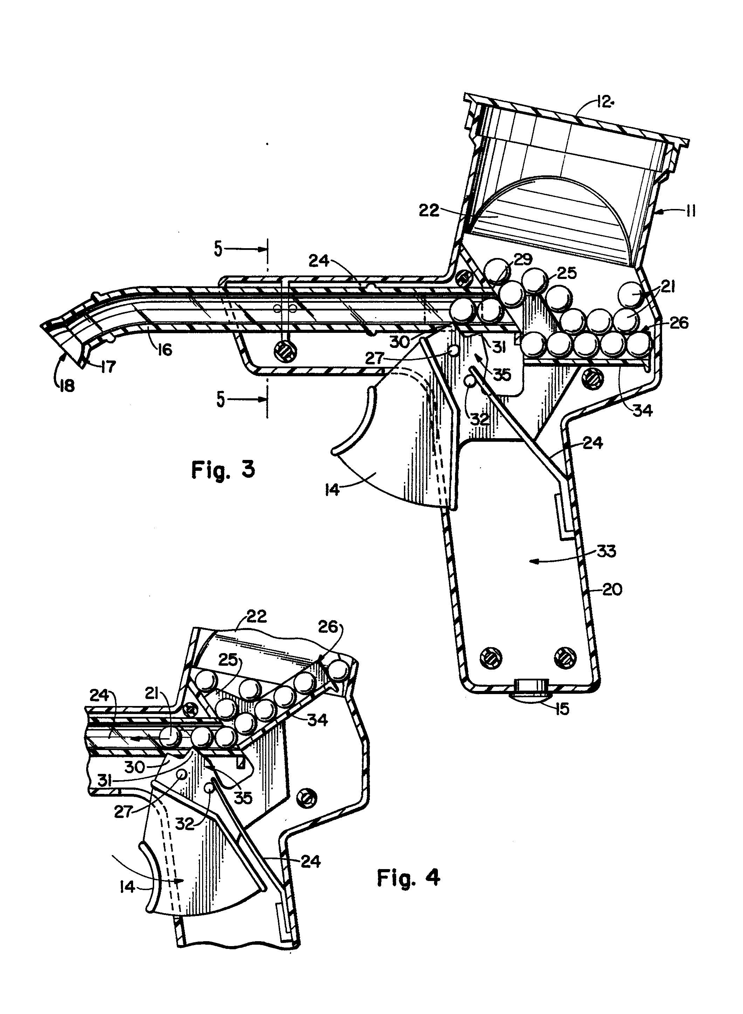

The body 10 of the bead dispenser has a cavity and includes a handle-shaped portion 20 and a bead chamber 11. The bead chamber stores a supply of beads 21 to be dispensed. Beads are introduced into the bead chamber through an opening in the top 12 of the bead chamber. A removable cap 28 is provided for closing the opening in the top of the bead chamber. The bead chamber has a funnel shaped portion 22 for aiding the flow of beads to the dispensing mechanism illustrated in detail in Fig. 4. The entire device may be made wholly of plastic. In the preferred embodiment, a translucent plastic is used for the body of the bead dispenser while the barrel member 16 is made of transparent plastic.

Cut outs 13 are provided in the body of the bead dispenser as an aid to viewing beads in the vicinity of the dispensing mechanism.

In many applications, beads to be dispensed come packed in a liquid solution. A drainage chamber 33 is provided within the handle 20 of the bead dispenser below and in communication with the bead chamber 11 to act as an area into which excess liquid solution may flow and be stored. A drain plug 15 is provided in the bottom of the drainage chamber to facilitate the removal of liquid solution from the bead dispenser.

Figure 3 illustrates the bead dispenser when the trigger member 14 is in the first trigger position. Rigidly attached to the trigger member is a dispensing member 35 which comprises a first gate member 31 and a second gate member 30. When the trigger member is in the first trigger position as in Fig. 3, the second gate member 30 impedes the travel of beads 21 down the barrel member 16 toward the outboard end 18 of the barrel member. When the trigger member is in the first trigger position, the first gate member 31 is withdrawn from the barrel and does not impede the travel of beads down the barrel member toward the outboard end 18 of the barrel member. When, as in Fig. 4, the trigger member is in the second trigger position, the first gate member 31 is moved into position to impede the travel of beads down the barrel member toward the outboard end 18 of the barrel member and the second gate member 30 is withdrawn from the barrel member releasing any bead that was located between the first gate member and the second gate member so that it can then travel down the tube toward the outboard end 18 of the barrel member for release into the reaction vessel 19. Biasing is applied to return the trigger member to the first trigger position. One end of a biasing member 24 rests slidably against a raised peg 32 which is connected rigidly to trigger member 14. The other end of the biasing member is attached rigidly to the body.

The trigger member 14 is attached rigidly to guide member 34. Guide member 34 has a guide slot 26. Beads from the bead chamber flow down toward the guide slot and are lined up in the guide slot for dispensing. Beads flow from the position in the guide slot closest to the first gate member 31 into the inboard end 29 of the barrel member. Beads not lined up in the guide slot are prevented from blocking the entrance to the barrel member by agitator member 25 which extends into the bead chamber, is attached rigidly to the guide member and which, when raised by actuating of the trigger member acts to push excess beads up in the bead chamber and away from the opening 29 thus allowing correctly aligned beads from the guide slot to flow into the barrel member.

Figure 5 shows a generally octagonally cross-sectioned barrel member 16 used to minimize the contact area of the bead 21 with the inside of the barrel member in order to facilitate the flow of beads down the barrel.

Thus, a diagnostic bead dispenser is disclosed which is useful for dispensing beads into reaction vessels. While embodiments and applications of this invention have been shown and described, it would be apparent to those skilled in the art that many more modifications are possible without departing from the spirit of the invention. The description, therefore, is provided to illustrate the invention and should not be construed to be limiting.

**Claims**

1. An apparatus useful for dispensing diagnostic assay beads, which comprises in combination a body having a cavity, a bead chamber within said cavity, a barrel member having a first end and a second end, said barrel member defining a passage, said first end mounted within said cavity and in communication with said bead chamber, said second end extending outwardly from said body and including an orifice; a dispensing member comprising a first gate member and a second gate member, said first gate member positioned apart from said second gate member to allow a single bead to occupy the space between said first gate member and said second gate member; a trigger member projecting form said body and operatively connected to said body, said trigger member operatively connected to said dispensing member, said trigger member having a first trigger position and a second trigger position wherein when said trigger member is in said first trigger position said first gate member does not impede the travel of beads within said barrel member toward said orifice and said second gate member impedes the travel of beads within said barrel member toward said orifice; and wherein when said trigger member is in said second trigger position said first gate member impedes the travel of beads within said barrel member toward said orifice and said second gate member does not impede the travel of beads within said barrel member toward said orifice; biasing means biasing said trigger member toward said first trigger position.

2. An apparatus as claimed in claim 1 additionally comprising a drainage chamber located below and connected to said bead chamber.

3. An apparatus as claimed in claim 1 or 2 wherein the orifice of said barrel member is flared.

4. An apparatus as claimed in claim 3 wherein said dispensing member is rigidly attached to said trigger member.

5. An apparatus as claimed in any one of claims 1 to 4 wherein said barrel member is curved so as to point generally downward.

6. An apparatus as claimed in claim 5 additionally comprising an agitator member operatively connected to said trigger member, said agitator member extending into said bead chamber.

7. An apparatus as claimed in claim 6 additionally comprising a guide member.

8. An apparatus as claimed in claim 7 wherein said guide member is operatively associated with said trigger member.

9. An apparatus as claimed in claim 8 wherein said guide member is rigidly attached to said trigger member.

10. An apparatus as claimed in claim 9 wherein said barrel member has an interior cross section of a generally octagonal shape.

0 209 994

Fig. 1

Fig. 5

Fig. 2

Fig. 3

Fig. 4

European Patent Office

**EUROPEAN SEARCH REPORT**

Application number

EP 86 30 4909

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 277 563 (ABBOTT LABORATORIES) * Claims; page 2, lines 21-36; page 3, lines 1-18; figures 2-7 * | 1 | G 01 N 33/48 G 01 N 33/543 A 61 J 7/00 |
| A | DE-A-3 403 264 (OLYMPUS OPTICAL CO.) | | |
| A | EP-A-0 071 256 (HOECHST-ROUSSEL PHARMACEUTICALS INC.) | | |
| A | CA-A-1 167 421 (M. BERGERON) | | |

---

| | | | TECHNICAL FIELDS SEARCHED (Int. Cl.4) |
|---|---|---|---|
| | | | G 01 N 33/00 A 61 J A 01 K |

The present search report has been drawn up for all claims

| Place of search THE HAGUE | Date of completion of the search 14-10-1986 | Examiner MEYLAERTS H. |
|---|---|---|

EPO Form 1503 03 82

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document